(19)

Europäisches Patentamt

European Patent Office

Office européen des brevets

(11) **EP 2 498 724 B1**

(12) **EUROPÄISCHE PATENTSCHRIFT**

(45) Veröffentlichungstag und Bekanntmachung des Hinweises auf die Patenterteilung:
**05.11.2014 Patentblatt 2014/45**

(21) Anmeldenummer: **10782546.5**

(22) Anmeldetag: **12.11.2010**

(51) Int Cl.:
**A61F 2/64** *(2006.01)* **A61F 2/68** *(2006.01)*

(86) Internationale Anmeldenummer:
**PCT/EP2010/006891**

(87) Internationale Veröffentlichungsnummer:
**WO 2011/057790 (19.05.2011 Gazette 2011/20)**

(54) **VERFAHREN UND VORRICHTUNG ZUR STEUERUNG EINES KÜNSTLICHEN ORTHETISCHEN ODER PROTHETISCHEN GELENKES**

METHOD AND DEVICE FOR CONTROLLING AN ARTIFICIAL ORTHOTIC OR PROSTHETIC JOINT

PROCÉDÉ ET DISPOSITIF POUR COMMANDER UNE ARTICULATION ORTHÉTIQUE OU PROTHÉTIQUE ARTIFICIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2009 DE 102009052888**

(43) Veröffentlichungstag der Anmeldung:
**19.09.2012 Patentblatt 2012/38**

(73) Patentinhaber: **Otto Bock Healthcare Products GmbH**
**1070 Wien (AT)**

(72) Erfinder:
• **SEYR, Martin**
  **A-1040 Wien (AT)**

• **KAMPAS, Philipp**
  **A-1020 Wien (AT)**
• **PAWLIK, Roland**
  **A-1130 Wien (AT)**
• **KALTENBORN, Sven**
  **37115 Duderstadt (DE)**

(74) Vertreter: **Stornebel, Kai et al**
**Gramm, Lins & Partner GbR**
**Theodor-Heuss-Strasse 1**
**38122 Braunschweig (DE)**

(56) Entgegenhaltungen:
**EP-A1- 1 570 817          DE-A1- 19 859 931**
**DE-T2- 60 309 685          US-A1- 2007 027 555**
**US-A1- 2008 228 287**

EP 2 498 724 B1

**Beschreibung**

Die Erfindung betrifft ein Verfahren und eine Vorrichtung zur Steuerung eines künstlichen orthetischen oder prothetischen Gelenkes einer unteren Extremität mit einer Widerstandseinrichtung, der zumindest ein Aktuator zugeordnet ist, über den der Beuge-und/oder Streckwiderstand in Abhängigkeit von Sensordaten verändert wird, wobei während der Benutzung des Gelenkes über Sensoren Zustandsinformationen bereitgestellt werden.

Eine sinnvolle prothetische Versorgung geriatrischer Patienten erfordert eine Orientierung an den Bedürfnissen der Patienten, der jeweiligen Aktivitäten und den medizinischen Notwendigkeiten. Häufig steht das Bedürfnis nach Sicherheit im Vordergrund, so dass während des Stehens eine Sperre des Kniegelenkes gewünscht wird. Die Sperre soll nach Möglichkeit belastungsabhängig und winkelabhängig aktivierbar sein und in jeder Stehsituation das Gefühl der Stabilität hervorrufen, da die Koordinationsfähigkeit, Beweglichkeit und die physischen Kräfte solcher Patienten mitunter eingeschränkt sein können.

Sofern der Patient mobil ist, soll während des Gehens in der Standphase ein hoher Flexionswiderstand vorhanden sein, um ein ungewolltes Einbeugen zu vermeiden, da ein Einbeugen häufig nicht schnell genug durch eine Hüftstreckung ausgeglichen werden kann.

Das Sitzen hingegen erfordert einen niedrigen Widerstand sowohl in Extensions- als auch Flexionsrichtung, damit der Patient sich ungehindert bewegen kann.

Es kann auch notwendig sein, eine Schwungphasenauslösung bereitzustellen, um das Gehen für den Patienten angenehmer zu machen.

Künstliche Gelenke, insbesondere Kniegelenke, für Orthesen oder Prothesen weisen ein oberes Anschlussteil und ein unteres Anschlussteil auf, die über eine Gelenkeinrichtung miteinander verbunden sind. An dem oberen Anschlussteil sind im Falle eines Kniegelenkes Aufnahmen für einen Oberschenkelstumpf oder eine Oberschenkelschiene angeordnet, während an dem unteren Anschlussteil ein Unterschenkelschaft oder eine Unterschenkelschiene angeordnet ist. Im einfachsten Fall ist das obere Anschlussteil mit dem unteren Anschlussteil durch ein einachsiges Gelenk verschwenkbar miteinander verbunden. Eine solche Anordnung ist nur in Ausnahmefällen ausreichend, um den gewünschten Erfolg, beispielsweise eine Unterstützung bei dem Einsatz einer Orthese oder ein natürliches Gangbild bei dem Einsatz in einer Prothese, zu gewährleisten.

Um die unterschiedlichen Anforderungen während der verschiedenen Phasen eines Schrittes oder bei anderen Verrichtungen möglichst natürlich darzustellen oder zu unterstützen, sind Widerstandseinrichtungen vorgesehen, die einen Flexionswiderstand oder einen Extensionswiderstand bereitstellen. Über den Flexionswiderstand wird eingestellt, wie leicht das untere Anschlussteil gegenüber dem oberen Anschlussteil in Flexionsrichtung verschwenkbar ist. Bei einem Kniegelenk wird daher über den Flexionswiderstand eingestellt, wie leicht der Unterschenkelschaft oder die Unterschenkelschiene im Verhältnis zu dem Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und kann einen Streckanschlag ausbilden. Bei anderen Gelenktypen, wie dem Hüftgelenk oder dem Knöchelgelenk, gelten diese Ausführungen entsprechend den kinematischen Verhältnissen.

Über einstellbare Widerstandseinrichtungen ist es möglich, den jeweiligen Flexions- und/oder Extensionswiderstand an den Nutzer der prothetischen oder orthetischen Einrichtung anzupassen oder auf verschiedene Gang- oder Bewegungssituationen einzugehen, um bei sich verändernden Bedingungen einen angepassten Widerstand bereitstellen zu können.

Aus der DE 10 2008 008 284 A1 ist ein orthopädietechnisches Kniegelenk mit einem O-berteil und einen verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von den ermittelten Sensordaten eingestellt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momenten-armes Anheben des Prothesenfußes detektiert und die Flexionsdämpfung in einer Anhebephase auf unterhalb eines Niveaus, das für eine Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2007 053 389 A1 beschreibt ein Verfahren und eine Vorrichtung zur Steuerung eines orthopädischen Gelenkes einer unteren Extremität mit zumindest einem Freiheitsgrad mit einem verstellbaren Aktuator zur Anpassung einer orthopädischen Einrichtung, die oberseitige Anschlussmittel an eine Gliedmaße und ein distal zu Anschlussmitteln gelenkig angeordnetes orthopädisches Gelenk aufweist, an Gehsituationen, die von einem Gehen in der Ebene abweichen. Dabei werden mehrere Parameter der orthopädischen Einrichtung über Sensoren erfasst, die erfassten Parameter mit Kriterien, die anhand mehrerer Parameter und/oder Parameterverläufe erstellt worden und in einer Rechnereinheit abgelegt sind, verglichen und ein Kriterium ausgewählt, das anhand der ermittelten Parameter oder Parameterverläufe geeignet ist. Auf der Grundlage des gewählten Kriteriums werden Beugungswiderstände, Bewegungsumfänge, Antriebskräfte und/oder deren Verläufe eingestellt, um Sonderfunktionen zu steuern, die vom Gehen in der Ebene abweichen. Ein Kippwinkel eines Teils der orthopädischen Einrichtung im Raum und/oder ein Verlauf einer Kippwinkeländerung eines Teils der orthopädischen Einrichtung können als Parameter herangezogen werden.

Weiterhin sind aus dem Stand der Technik so genannte Bremskniegelenke bekannt, bei denen mechanisch bei wachsender Axialbelastung der Flexions- und Extensionswiderstand erhöht wird. Dies wird im einfachsten Fall dadurch erreicht, dass zwei Bremsflächen vorgesehen sind, die durch eine Bodenreaktionskraft aufeinander gepresst werden. Eine solche Ausgestaltung ist an der Bremseinrichtung ist für moderne Prothesenkniegelenke mit gesteuerten Widerstandseinrichtungen nicht einsetzbar.

Es hat sich bewährt, dass Kniegelenke in der Standphase während des Gehens oder während des Stehens einen hohen Widerstand bieten, wobei das Gelenk nicht vollständig gesperrt wird. Bei einem vollständig gestreckten Kniegelenk wird das Beugen des Gelenks dadurch verhindert, dass der Kraftvektor vor der Gelenksachse liegt und somit das Gelenk in den Streckanschlag gedrückt wird. Sobald der Kraftvektor hinter die Gelenkachse wandert, besteht die Gefahr, dass das Gelenk einknickt. Daher ist es notwendig, in einer leicht gebeugten Stellung ebenfalls einen erhöhten Widerstand bereitzustellen. Dass das Gelenk in einer leicht gebeugten Stellung nicht vollständig sperrt, hat den Vorteil, dass der Nutzer des Gelenks noch Eingriffsmöglichkeiten in die Gelenksbewegung hat. Sollte er z.B. auf einer Treppe stehen und das Gleichgewicht verlieren, würde er dann über ein gesperrtes Gelenk unkontrolliert stürzen, während er ein Gelenk mit einem hohen Flexionswiderstand mittels der Stumpfkraft noch beugen und damit die Folgen des Sturzes vermindern oder das Stürzen ganz verhindern kann. Ebenfalls erleichtert eine hohe Dämpfung während des Stehens das Manövrieren des Gelenkes in engen Räumen oder das Niedersetzen.

Wenn das Gelenk nur einen hohen Widerstand bietet und nicht vollständig blockiert, ist das Belasten der Prothese z.B. beim Stehen auf geneigten Flächen nicht möglich, wenn der Bodenreaktionskraftvektor zu weit zur Ferse wandert und damit nicht mehr vor der Knieachse, sondern hinter der Knieachse liegt und dadurch das Knie beugt. Auch ist das Stehen auf einem gebeugten Knie nicht möglich, weil durch die Kniebeugung die Kniegelenksachse vor den Bodenreaktionskraftvektor wandert und dadurch das Knie weiter gebeugt wird.

Weiterhin sind aus dem Stand der Technik Einrichtungen bekannt, bei denen ein gesonderter Modus eingestellt werden muss, um ein blockiertes Stehen auf einer Rampe oder auf einem gebeugten Knie zu aktivieren. In einem solchen Modus kann das Gelenk nicht weiter als bis zu einem einstellbaren Winkel gebeugt werden. Zum Weitergehen oder Hinsetzen muss bewusst in einen anderen Modus gewechselt werden.

Die DE 603 09 685 T2 beschreibt ein prothetisches Gelenksystem, insbesondere ein Fußknöchel- und Fußgelenksystem, das die natürliche menschliche Fortbewegung und die menschliche Biomechanik über Sensorfeedback, Zeitfeedback, eine elektronisch gesteuerte Dämpfungsgelenkeinheit und ein Mikroprozessorsteuersystem simuliert. Das Feedbacksensorsystem liefert dem Dämpfungssystem Informationen, beispielsweise in Bezug auf die Gewichtsverteilung, Aufprallzeiten oder dergleichen, um den Gangzyklus des Benutzers zu ermitteln und automatisch die Operationen der Gelenkeinheit zu steuern. Das Sensorsystem kann auch einen Dehnungssensor, Momentensensor, Drucksensor oder ähnliches aufweisen, der mit einer Mikroprozessoreinheit sowie einer Stromquelle in Verbindung stehen kann. Es können Druck-/Kraft- Formänderungssensoren im Dämpfungssystem angeordnet sein, um die auf einen Fersenabschnitt und/oder Zehenabschnitt einwirkenden Kräfte zu ermitteln.

Die US 2008/0228287 A1 betrifft ein Prothesenbein mit einem Hydraulikzylinder zum Einstellen eines Beuge- und Streckwiderstandes eines Kniegelenkes. Über ein Bodenkontaktermittlungsbauteil werden Ventile in der Hydraulikeinheit geschlossen, wenn der Prothesennutzer mit der Ferse oder mit dem Vorderfußbereich auftritt. In Abhängigkeit von dem Beugewinkel kann ein Bypasskanal geschlossen werden.

Aufgabe der vorliegenden Erfindung ist es, ein Verfahren einer Vorrichtung bereitzustellen, mit denen es möglich ist, das Knie automatisch in bestimmten Situationen mit einem erhöhten Widerstand zu belasten oder zu blockieren, ohne dass eine bewusste Aktivierung oder Deaktivierung des Modus erfolgen muss.

Erfindungsgemäß wird diese Aufgabe mit einem Verfahren gemäß dem Hauptanspruch und einer Vorrichtung gemäß dem nebengeordneten Anspruch erreicht. Vorteilhafte Ausgestaltungen und Weiterbildungen der Erfindung sind in den Unteransprüchen aufgeführt.

Das erfindungsgemäße Verfahren zur Steuerung eines künstlichen orthetischen oder prothetischen Kniegelenkes mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Kniegelenkes eine Steuereinrichtung über Sensoren Zustandsinformationen bereit gestellt werden, sieht vor, dass der jeweilige Widerstand auf der Grundlage empfangener Sensordaten und der Auswertung der Sensordaten verändert wird, indem der Aktuator entsprechend der Auswertung aktiviert wird, wobei der Widerstand in der Standphase oder während des Stehens von einem Ausgangswert in Abhängigkeit von der Bodenreaktionskraft bis zu einer Sperre des Kniegelenkes erhöht wird. Nach der Detektierung der Standphase oder des Stehens wird bei Vorliegen einer vorbestimmten Bodenreaktionskraft der Gelenkwiderstand erhöht, bis zu einer Sperre des Gelenkes, um automatisch in einen sicheren oder gesicherten Zustand zu gelangen. Der Widerstand kann während der Standphase oder während des Stehens bei zunehmender Belastung kontinuierlich erhöht werden, wobei die Erhöhung vorteilhafter Weise nur dann durchgeführt wird, wenn ein Schwellwert, z.B. der Bodenreaktionskraft, erreicht oder überschritten wird. Die Bodenreaktionskraft kann dabei direkt gemessen werden. In den meisten Fällen wird allerdings nur eine aussagekräftige Komponente der Bodenreaktionskraft, wie z.B. die Komponente in Unterschenkelrichtung gemessen. Der Ausgangswiderstand kann einstellbar sein, so dass das Niveau, von dem aus der Widerstand erhöht und auf den er ggf. wieder reduziert wird, an den Patienten angepasst werden.

Um den Zustand des erhöhten Flexionswiderstandes nur dann einzusetzen, wenn die Gefahr eines Einknickens des Kniegelenkes besteht, ist vorgesehen, dass die Sperrung nur dann erfolgt, wenn der Bodenreaktionskraftvektor hinter der Knieachse verläuft, so dass bei einer weitergehenden Belastung in Axialrichtung des versorgten Beines eine zunehmende Beugung auftreten würde. Je nach Prothesen- oder Orthesenaufbau kann dies bei unterschiedlichen Beugewinkeln erfolgen. Besonders stabil aufgebaute Gelenke, bei denen der Bodenreaktionskraftvektor im gestreckten Zustand weit vor der Gelenkachse liegt, werden erst bei einem größeren Gelenkwinkel mit einem erhöhten Widerstand beaufschlagt, während weniger stabil aufgebaute Gelenke bereits vorher einen erhöhten Widerstand benötigen. Ein weniger stabiler und auch unsicherer Aufbau, z.B. durch eine Vorverlagerung des Kniegelenks, bringt, wenn durch die Steuerung das Einknicken des Kniegelenkes verhindert und die Prothese gesichert wird, dem Patienten Vorteile. Bei sonst gleichen Bedingungen führt die Vorverlagerung des Kniegelenkes zu einer schnelleren und größeren Kniebeugung beim Fersenauftritt und dämpft den Stoß. Weiterhin nimmt das hüftbeugende Moment zum Einleiten der Schwungphase und zur Beugung der Prothese in der Schwungphase ab, was positiv für den Patienten wirkt. Die Vorverlagerung des Kniegelenkes bewirkt auch eine Verkürzung der Prothesenlänge in der mittleren Schwungphase, was das Stolperrisiko für den Patienten reduziert. Somit wird der Widerstand auch in Abhängigkeit von dem Gelenkwinkel oder dem Inertialwinkel einer Gelenkkomponente, insbesondere des Unterschenkelteils und des Oberschenkelteils, erhöht, da auch bei einem gestreckten Kniegelenk und einem stabilen Aufbau beim Stehen auf einer schrägen Ebene der Bodenreaktionskraftvektor hinter der Kniegelenkachse liegen kann, was zu einem Einknicken des Kniegelenkes führen kann. Der Inertialwinkel ist vor allem hilfreich, um ein Blockieren während des Sitzens, also bei einem annähernd waagerechten Oberschenkel, zu verhindern.

Der Widerstand kann auch in Abhängigkeit von dem Abstand der Bodenreaktionskraft zu einem Bezugspunkt an einem Anschlussteil an dem Gelenk oder in Abhängigkeit von einem Moment um den Bezugspunkt erhöht oder gesperrt werden.

Um nach einer Erhöhung des Widerstandes bis zu einer Sperre die Gelenkeinrichtung für andere Aktivitäten als Stehen nutzen zu können, ist vorgesehen, dass der Widerstand in Abhängigkeit von einem Inertialwinkel, einer Änderung des Inertialwinkels und/oder der Inertialwinkelgeschwindigkeit eines Anschlussteiles des Gelenkes z.B. auf den Ausgangswert reduziert wird. Es wird also detektiert, dass ein Teil des Gelenkes bewegt wird, beispielsweise dass die Prothese entlastet wird oder der Nutzer über die Prothese nach vorne oder hinten abrollt. Daraus wird erkannt, dass kein Stehen mehr vorliegt, so dass der Widerstand verringert werden muss.

Von Stehen spricht man, wenn der Prothesenfuß aufgesetzt ist und der Prothesenträger sich nicht in einer Vorwärts- oder Rückwärtsbewegung befindet. Eine Axiallast oder Bodenreaktionskraft wird auf die Prothese und den Prothesenfuß ausgeübt. Das Prothesenkniegelenk ist meistens gestreckt, wobei aber durch das vorliegende Verfahren auch ein Stehen mit gebeugtem Knie möglich sein soll. Dieses belastungsabhängige Verhalten wird bevorzugt als "Funktion" umgesetzt. Eine Funktion kann freigeschaltet oder gesperrt werden. Wenn sie freigeschaltet ist, wird sie immer aktiviert, wenn die Sensorkriterien zur Erhöhung des Widerstandes erfüllt sind. Sind die Kriterien nicht mehr erfüllt, kann das Gelenk auch in andere Steuerungszustände, wie zum Beispiel eine Schwungphasensteuerung, übergehen. Ist sie gesperrt, kann sie nicht mehr aktiviert werden. Die anderen Steuerungszustände werden davon aber nicht betroffen. Es ist aber auch möglich, die Steuerung als "Modus" umzusetzen.

Der Stehmodus ist ein Steuerungsverhalten, das separat aktiviert werden muss. Anders als eine Stehfunktion, die permanent vorhanden ist und abfragt, ob die Kriterien für die Stehfunktion erfüllt sind oder nicht, um dann eine entsprechende Anpassung des oder der Widerstände vorzunehmen, wird der Modus, im vorliegenden Verfahren der Stehmodus, über eine bewusst auszuführende Tätigkeit aktiviert. Ist das Verfahren als Modus umgesetzt, beschränkt sich die Funktion des Gelenks ausschließlich auf das beschriebene Verhalten. Ein Übergang in andere Steuerungszustände, wie zum Beispiel eine Schwungphasensteuerung, ist nur nach bewusster Deaktivierung des Modus möglich.

Ebenfalls ist es möglich, dass die Erhöhung des Widerstandes trotz Vorliegen der entsprechenden Bodenreaktionskraft und des Gelenkwinkels gar nicht eingeleitet wird, wenn eine Inertialgeschwindigkeit einer Gelenkkomponente vorhanden ist, also kein Stehzustand vorhanden ist. Ebenfalls kann in Abhängigkeit des Inertialwinkels eine Blockierung des Gelenkes ausgeschlossen werden, beispielsweise wenn das Oberschenkelteil in Gehrichtung nach vorne geneigt ist, also das proximale Ende des Oberschenkelteils in Gehrichtung vor dem distalen Ende des Oberschenkelteils liegt.

Zur Bestimmung des Inertialwinkels eines Anschlussteils ist es möglich, diesen unmittelbar oder aus dem Inertialwinkel eines anderen Anschlussteils in Verbindung mit einem entsprechenden Gelenkwinkel zu bestimmen.

Beim Entlasten des Gelenkes, beispielsweise beim Anheben des Beines, kann eine Hysterese vorgesehen werden, die den Widerstand erst bei einer niedrigeren Belastung wieder reduziert, also dass der Schwellwert wesentlich unterschritten werden muss, um eine Widerstandsreduzierung zu bewirken.

Um den Widerstand beim Weitergehen oder Hinsetzen wieder zu reduzieren, kann die Inertialwinkelgeschwindigkeit des Anschlussteils, der Kniewinkel, die Kniewinkelgeschwindigkeit, die Veränderung des Abstandes des Bodenreaktionskraftvektors zu einem Anschlussteil und/oder die Veränderung des Inertialwinkels eines Anschlussteils verwendet werden. So eignen sich zum Beispiel der Kniewinkel, die Kniewinkelgeschwindigkeit und die Inertialwinkelgeschwindigkeit gut, um ein Vorwärtsgehen zu erkennen. Weiterhin ist vorgesehen, dass die Sperrung der Flexion dann eingeleitet wird, wenn die Kniewinkelgeschwindigkeit Null oder nahezu Null ist, um sicherzustellen, dass das Kniegelenk nur während der Stillstandes gesperrt wird. Dieser Zustand kann eintreten, wenn der Kraftvektor hinter der Knieachse läge, der Patient vorübergehend, bis die Flexionssperre anspricht, die Beugung aber mit der Hüfte kompensiert, so dass das Kniemoment und die Kniewinkelgeschwindigkeit Null werden. Beim Stehen kann dann durch die aktivierte Flexionssperre das Kniemoment beugend sein, ohne dass das Knie einsinkt.

Der Abstand des Bodenreaktionskraftvektors zu einem Anschlussteil eignet sich gut, um ein Hinsetzen zu erkennen. Das Reduzieren des Widerstandes kann dabei bei Überschreiten eines Schwellwertes für die genannten Parameter diskret oder kontinuierlich erfolgen.

Die Reduzierung ebenso wie die Erhöhung des Widerstandes können durch mehrere Parameter eingeleitet werden, beispielsweise Belastung, Gelenkwinkel und Inertialwinkel, wobei verschiedene Formfunktionen vorliegen, über die durch eine Verknüpfung mehrerer Bedingungen ermittelt wird, ob und um welchen Faktor der Widerstand vergrößert wird bzw. verringert wird. Damit ergibt sich ein sanftes Sperren und Entsperren des Gelenkes in Abhängigkeit von mehreren Einflussgrößen.

Da ein Blockieren des Gelenkes im Sitzen mitunter unerwünscht ist, beispielsweise um ein gesperrtes Kniegelenk und ein verkeilendes Gelenk beim Autofahren zu verhindern, ist vorgesehen, dass der Widerstand nicht bis zu einer Sperre erhöht werden kann, wenn der Oberschenkelteil annähernd waagerecht ist. Dazu wird der Inertialwinkel des Oberschenkelteils ermittelt. Die oben beschriebene Erhöhung des Widerstandes bis zu einer Blockade des Gelenkes kann Teil einer Gesamtsteuerung eines Gelenkes sein, möglich ist auch, dass sie die einzige Funktion eines Gelenkes ist. Sie kann dabei einen bewusst zu aktivierenden Modus bilden, der über eine Einstelleinrichtung aktiviert oder deaktiviert werden kann. Es ist ebenfalls möglich, dass diese Funktion latent vorhanden ist, so dass während der Standphase oder des Stehens in dem normalen Steuerprogramm eines Kniegelenkes diese Funktion jederzeit vorhanden ist, wenn die Voraussetzungen erfüllt sind, um das Gelenk zu sperren.

Die Vorrichtung zum Durchführen des Verfahrens, wie es oben beschrieben ist, sieht eine einstellbare Widerstandseinrichtung vor, die zwischen zwei gelenkig aneinander gelagerten Komponenten eines künstlichen orthetischen oder prothetischen Gelenkes angeordnet ist, die eine Steuereinrichtung und Sensoren aufweist, die Zustandsinformationen der Vorrichtung erfasst. Weiterhin ist eine Einstellvorrichtung vorgesehen, über die die belastungsabhängige Widerstandsänderung aktivierbar und/oder deaktivierbar ist.

Nachfolgend wird ein Ausführungsbeispiel der Erfindung anhand der beigefügten Figuren näher erläutert. Es zeigen:

Figur 1    einen Widerstandsverlauf;

Figur 2    Kriterien zur Steuerung der Widerstandsveränderung;

Figur 3    eine schematische Darstellung einer Prothese;

Figur 4    eine schematische Darstellung zur Berechnung eines Abstandes;

Figur 5    eine schematische Darstellung zur Berechnung eines Abstandes auf der Grundlage mehrerer Sensorwerte; sowie

Figur 6    eine Konvention der Winkelangaben.

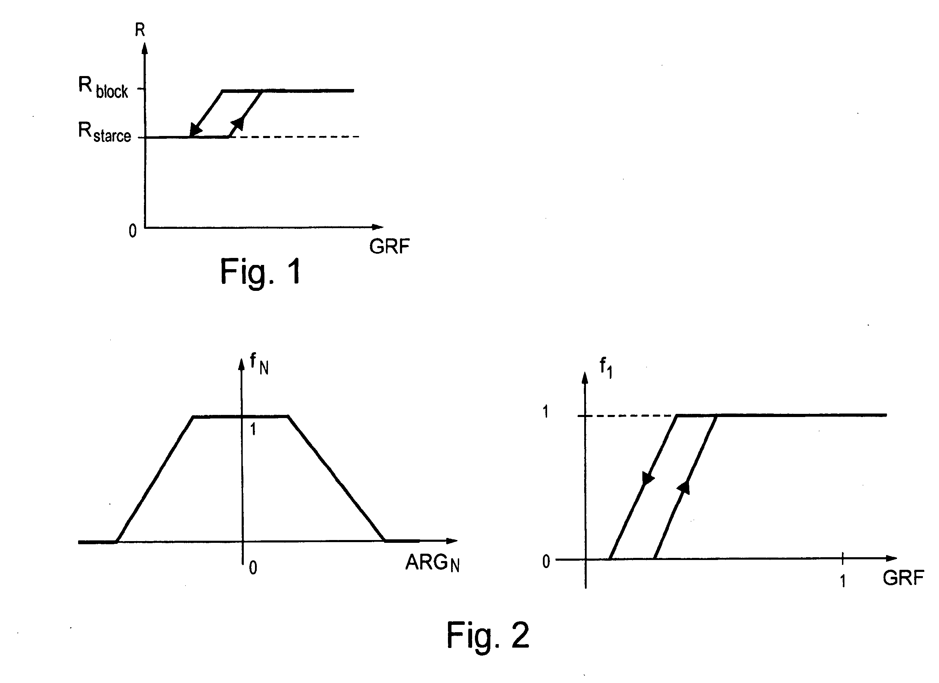

In Figur 1 ist der Verlauf des Widerstandes R bzw. der Widerstandsveränderung über die Belastung durch die Bodenreaktionskraft GRF dargestellt. In der Standphase oder während des Stehens wird der normale Standphasenwiderstand $R_{stance}$ eingestellt, um während des Gehens ein möglichst natürliches Gangbild mit einer Prothese oder Orthese zu erhalten. Wird ein Schwellwert der Bodenreaktionskraft GRF überschritten, wird der Widerstand R angehoben, wobei während der Standphase bei zunehmender Belastung durch die Bodenreaktionskraft GRF eine kontinuierliche Erhöhung des Widerstandes R bis auf ein Blockadewiderstand $R_{block}$ erreicht wird. Dieser Blockierwiderstand $R_{block}$ verhindert eine Flexion des Kniegelenkes wirksam, ist jedoch vorteilhafterweise so bemessen, dass keine mechanische Schädigung bei Überschreiten einer Belastungsgrenze für einzelne Komponenten der Prothese oder Orthese auftreten. Wird dann im weiteren Verlauf die Bodenreaktionskraft GRF wieder reduziert, ist vorgesehen, dass der Widerstand von dem Blockierwert $R_{block}$ erst dann wieder reduziert wird, wenn die Belastung unterhalb des Schwellwertes angelangt ist, der erreicht wurde, um eine Widerstandserhöhung zu veranlassen. Nach Unterschreiten dieses Schwellwertes wird dann der Widerstand R auf das normale Standphasenniveau reduziert, bis der Standphasenwiderstand $R_{stance}$ erreicht ist.

Die Art und Weise, wie der einzustellende Widerstand bestimmt werden kann, ist unterschiedlich, in der Figur 2 sind Formfunktionen dargestellt, die als Faktoren zur Berechnung des Widerstandes R eingesetzt werden können. Die rechte Kurve zeigt dabei die Funktion für die Belastung durch die Bodenreaktionskraft GRF, die normiert ist. Die linke Kurve zeigt, wie weitere Funktionen $f_N$ in die Berechnung des Sollwiderstandes eingehen können. Als Argumente für diese Funktionen können Indikatoren für eine Bewegung des Gelenkes, wie die Kniewinkelgeschwindigkeit, die Inertialwinkelgeschwindigkeit, die Abweichung des Abstandes der Bodenreaktionskraft von einem Gelenkteil vom Zeitpunkt des Eintritts der Sperre oder die Abweichung des Inertialwinkels eines Gelenkteils vom Eintritt der Sperre dienen. Alle Funktionen $f_1$ bis $f_N$ können durch Multiplikation zum Sollwert des Widerstandes verknüpft werden, indem sie z.B. die Differenz von einem Standphasenwiderstand zu einem Blockierwiderstand gewichten und so bestimmen, um welches Maß der Standphasenwiderstand $R_{stance}$ erhöht wird. Der Maximalwert für den Blockierwiderstand $R_{block}$ verringert um den Wert für den normalen Standphasenwiderstand $R_{stance}$ wird mit den beiden Argumenten $f_1$ und $f_N$ multipliziert. Ist eine Funktion Null, bleibt der Standphasenwiderstand $R_{stance}$ unverändert, sobald alle Funktionen $f_1$ bis $f_N$ größer als 0 sind, wird der Standphasenwiderstand $R_{stance}$ erhöht oder, wenn die Funktionen kleiner werden, der erhöhte Widerstand reduziert. Der Widerstand R errechnet sich daher aus

$$R = R_{stance} + (R_{block} - R_{stance}) * f_1(ARG_1) * f_2(ARG_2) * \ldots + f_N(ARG_N)$$

Es kann nach dem Reduzieren des Widerstandes nach dem Aktivieren der Sperre auch ein Set von Funktionen $f_N$ verwendet werden, das von demjenigen verschieden ist, was zum Aktivieren der Sperre eingesetzt wird.

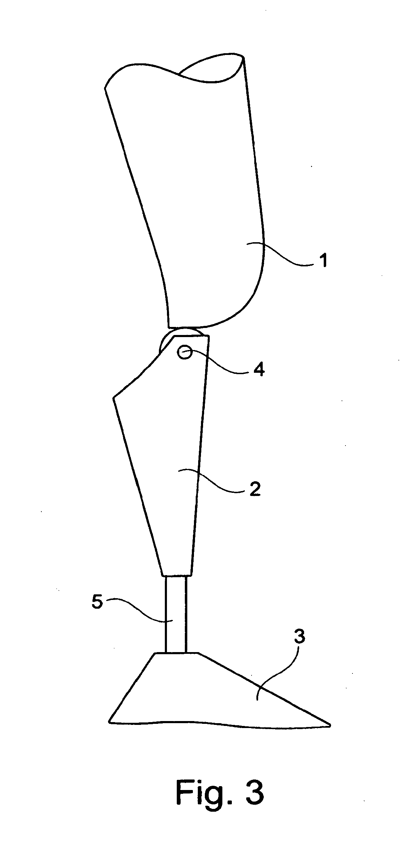

In der Figur 3 ist in einer schematischen Darstellung einer Beinprothese mit einem Oberschenkelschaft 1 zur Aufnahme eines Oberschenkelstumpfes gezeigt. Der Oberschenkelschaft 1 wird auch als oberes Anschlussteil bezeichnet. An dem oberen Anschlussteil 1 ist ein unteres Anschlussteil 2 in Gestalt eines Unterschenkelschaftes mit einer Widerstandseinrichtung angeordnet. An dem unteren Anschlussteil 2 ist ein Prothesenfuß 3 angeordnet. Das untere Anschlussteil 2 ist an dem oberen Anschlussteil 1 über ein Gelenk 4 schwenkbar befestigt. In dem Gelenk 4 ist ein Momentensensor angeordnet, der das wirksame Kniemoment ermittelt. In dem unteren Anschlussteil 2 ist ein Verbindungsteil 5 zu dem Prothesenfuß 3 vorgesehen, in dem eine Einrichtung zur Ermittlung der wirksamen Axialkraft sowie des Knöchelmomentes untergebracht sind. Es ist möglich, dass nicht alle Sensoren in einer Beinprothese vorhanden sind, ggf. kann auf einen Knöchelmomentsensor oder den Kniemomentsensor verzichtet werden.

In dem unteren Anschlussteil 2 befindet sich neben der Widerstandseinrichtung, die den Beuge- und Streckwiderstand bereitstellt, eine Steuereinrichtung, über die es möglich ist, den jeweiligen Widerstand auf der Grundlage der empfangenen Sensordaten und der Auswertung der Sensordaten zu verändern, indem ein Aktuator entsprechend der Auswertung aktiviert wird und die Widerstandseinrichtung so einstellt, dass der gewünschte oder erforderliche Widerstand in Extensions- und/oder Flexionsrichtung vorliegt. Dazu kann es vorgesehen sein, dass die Sensordaten zur Erzeugung zumindest einer Hilfsvariable verwendet werden, die über eine mathematische Verknüpfung zweier oder mehrerer Sensordaten erhalten wird. Dadurch ist es möglich, dass mehrere Kraft- oder Momentensensoren miteinander verknüpft werden, um Kräfte, Abstände und/oder Momente zu errechnen, die nicht unmittelbar im Bereich der Sensoren herrschen. So ist es beispielsweise möglich, Schnittkräfte, Schnittmomente oder Abstände in bestimmten Referenzebenen zu errechnen, um ausgehend davon beurteilen zu können, welche Funktionen gegenwärtig ausgeführt werden soll, damit ein möglichst natürliches Gangbild erreicht werden kann. Als Funktion werden dabei diejenigen Steuerungsabläufe bezeichnet, die im Rahmen einer natürlichen Bewegung auftreten, ein Modus hingegen ist ein Steuerungszustand, der durch einen willkürlichen Akt eingestellt wird, beispielsweise durch Betätigung eines gesonderten Schalters oder durch eine bewusste, ggf. bewusst unnatürliche Abfolge von Bewegungen.

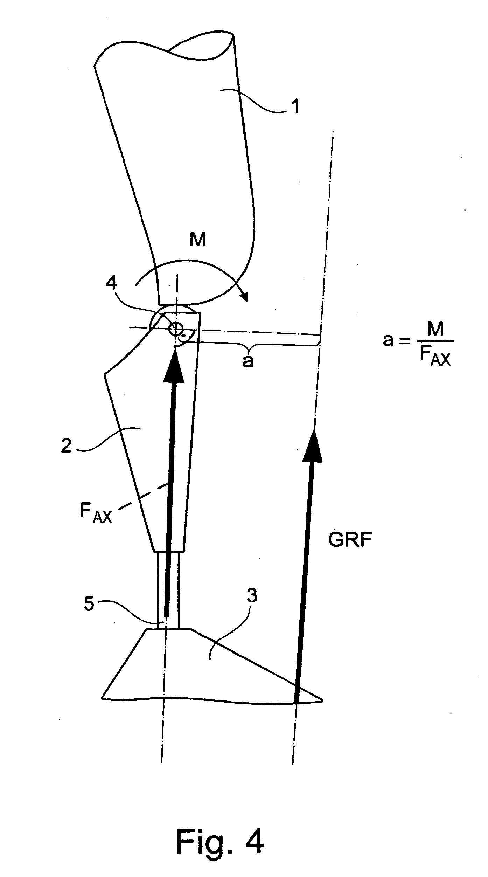

In der Figur 4 ist schematisch dargestellt, wie als Argument der Abstand a des Abstandes des Bodenreaktionskraftvektors GRF zu der Knieachse verwendet wird. Der Abstand a errechnet sich aus dem Quotienten des Kniemomentes M und der Axialkraft $F_{AX}$. Je größer das Kniemoment M im Verhältnis zur Axialkraft $F_{AX}$ ist, desto größer ist der Abstand a des Bodenreaktionskraftvektors GRF in der Referenzhöhe, die vorliegend die Knieachse bildet. Auf der Grundlage des Argumentes a ist es möglich, den Streckwiderstand und/oder den Beugewiderstand zu variieren, da über das Argument a errechnet werden kann, ob eine Stehen vorliegt oder das Stehen abgebrochen worden ist, sodass ausgehend davon ein vorher festgelegter Beuge- und/oder Streckwiderstand eingestellt wird. Durch die Veränderung des Arguments a kann bestimmt werden, wie die gegenwärtige Bewegung verläuft, sodass innerhalb der Bewegung, auch innerhalb der Standphase oder der Schwungphase, eine Anpassung des Streck- und/oder Beugewiderstandes erfolgen kann. Die Veränderung der Widerstände erfolgt bevorzugt kontinuierlich und in Abhängigkeit von der Veränderung dem Argument oder den Argumenten.

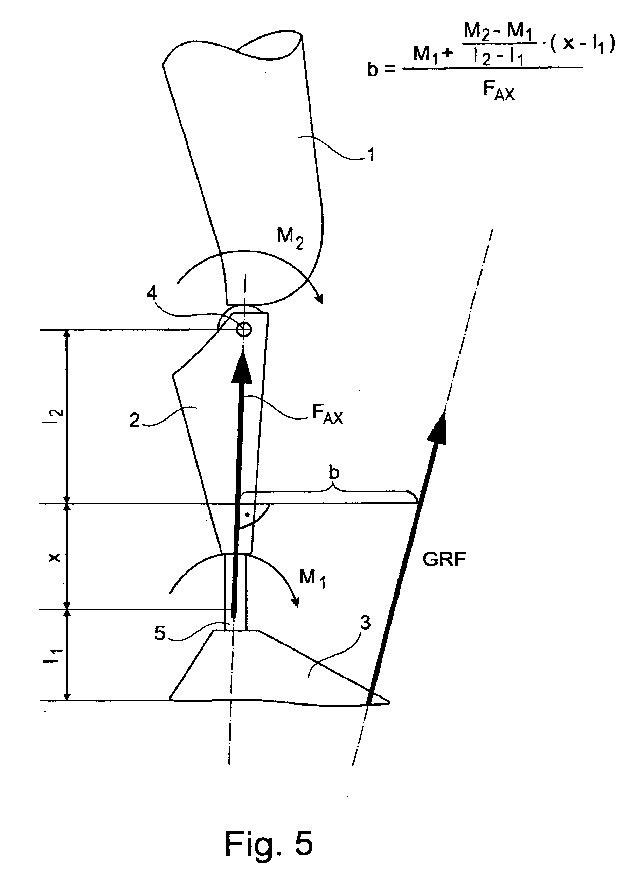

In der Figur 5 ist dargestellt, wie das Argument b in Gestalt des Abstandes des Bodenreaktionskraftvektors GRF in einer Referenzhöhe zu der Verbindungslinie der Momentensensoren berechnet werden kann. Das Argument b errechnet sich aus

$$b = \frac{M1 + \dfrac{M2 - M1}{l2 - l1} * (x - l1)}{FAX}$$

wobei $M_1$ das wirksame Moment im Verbindungsteil 5, in der Regel das Knöchelmoment in der Höhe $l_1$ vom Fußboden ist, das Moment $M_2$ das Kniemoment in Höhe der Knieachse 4 ist, die in einem Abstand von $l_2$ zum Fußboden liegt. Die Größe x ist die Referenzhöhe, die Kraft $F_{AX}$ ist die innerhalb des Verbindungsteils 5 bzw. in dem unteren Anschlussteil 2 wirksame Axialkraft. Über die Veränderung des Arguments b ist es, wie vorgeschrieben, möglich, kontinuierlich, sowohl während der Schwungphase als auch während der Standphase, die jeweiligen Widerstände einzustellen und an die vorhandenen Veränderungen anzupassen. Dadurch ist es möglich, verschiedene Funktionen zu aktivieren, die automatisch erkannt werden, beispielsweise eine Stehfunktion, über die beispielsweise verhindert wird, dass das Kniegelenk ungewollt einknickt.

Die Erhöhung des Widerstandes, insbesondere des Flexionswiderstandes während der Standphase oder während des Stehens, kann als eine latente Funktion, die ständig zur Verfügung steht, umgesetzt werden. Befindet sich der Patient in der Standphase oder steht er, wird automatisch der Widerstand gegen eine Flexion erhöht, bis zur Blockade des Gelenkes, insbesondere des Kniegelenkes. Dabei wird auch der Kniewinkel berücksichtigt. Befindet sich beispielsweise das Knie in einer gestreckten Stellung, ist aufgrund des in der Regel stabilen Aufbaues keine Sperrung der Flexionsbewegung notwendig. Befindet sich das Kniegelenk jedoch in einer leicht gebeugten Stellung, beispielsweise größer als 4° zwischen der Längserstreckung des Unterschenkelteils und des Oberschenkelteils, und tritt dann noch eine Belastung seitens einer Bodenreaktionskraft hinzu, dann wird das Kniegelenk automatisch gesperrt, da anzunehmen ist, dass trotz der Kniebeugung keine Flexion gewünscht ist.

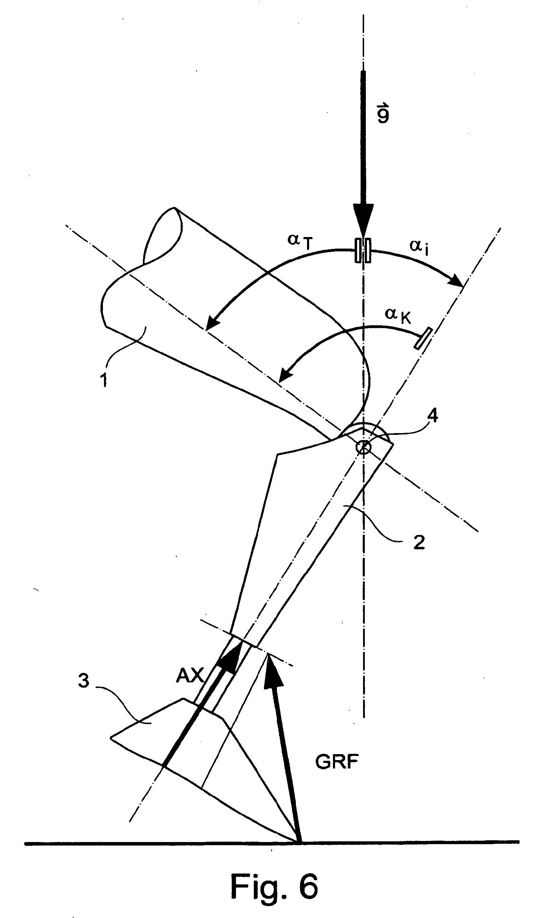

In der Figur 6 ist schematisch eine Prothese in sitzender Position dargestellt. Befindet sich der Prothesennutzer in einer sitzenden Position, ist es angenehm, wenn der Extensionswiderstand und der Flexionswiderstand der Widerstandseinrichtung gering sind, so dass die während des Sitzens ausgeführten Bewegungen, die in der Regel einen geringen Bewegungsumfang aufweisen, ohne Beeinträchtigungen durchgeführt werden können.

Um die Widerstandsveränderungen und eine Zustandsermittlung automatisch durchführen zu können, ist es vorgesehen, dass der Inertialwinkel $\alpha_T$ und/oder der Kniewinkel $\alpha_K$ gemessen werden. Der Inertialwinkel $\alpha_T$ des Oberschenkelteils 1 wird zur Vertikalen gemessen, die in Schwerkraftrichtung wirkend angenommen wird. In der Figur 6 ist dies durch den Schwerkraftvektor g angedeutet. Als Bezugsgröße für den Inertialwinkel $\alpha_T$ wird die Längsachse des Oberschenkelteils 1 angenommen, die durch die Schwenkachse des Prothesenkniegelenkes 4 geht. Dabei entspricht die Längsachse ungefähr der Orientierung eines natürlichen Oberschenkelknochens und erstreckt sich im Wesentlichen mittig zu dem Oberschenkelteil 1, das in der Regel als ein Oberschenkelschaft ausgebildet ist.

Der Kniewinkel $\alpha_K$ liegt zwischen der Längsstreckung des Unterschenkelteils 2 und der Längserstreckung des Oberschenkelteils 1 an. Auch hier geht die Längserstreckung des Unterschenkelteils 2 durch die Gelenkachse des Prothesenkniegelenkes 4. Der Kniewinkel $\alpha_K$ kann sich aus dem Inertialwinkel $\alpha_T$ des Oberschenkelteils 1 und dem Inertialwinkel $\alpha_i$ des Unterschenkelteils 2 errechnen lassen, wobei aufgrund der Berechnung der Inertialwinkel $\alpha_T$ und $\alpha_i$ ausgehend von dem Schwerkraftvektor g eine angepasste Vorzeichenregelung eingeführt wird, so dass der Inertialwinkel $\alpha_T$ des Oberschenkelteils 1 sich aus der Differenz zwischen dem Kniewinkel $\alpha_K$ und dem Inertialwinkel $\alpha_i$ des Unterschenkelteils 2 ergibt.

Darüber wird die Bodenreaktionskraft GRF bzw. die Axialkraft AX, die in Längsrichtung des Unterschenkelteils 2 wirksam ist, bestimmt, um auf Grundlage der vorliegenden Kräfte zu entscheiden, ob sich der Prothesennutzer in einer sitzenden oder stehenden Stellung befindet.

In der Regel reduziert sich die Bodenreaktionskraft GRF signifikant, wenn der Prothesennutzer sitzt. Unterschreitet also die Bodenreaktionskraft GRF einen Schwellwert, ist dies ein Faktor bei der Beurteilung, ob ein sitzender Zustand vorliegt. Wird eine große Bodenreaktionskraft GRF detektiert, kann eine stehende Stellung angenommen werden, ebenso kann ein Aufstehen angenommen werden, in beiden Fällen ist eine Erhöhung des Flexionswiderstandes sinnvoll, um bei einer hohen Bodenreaktionskraft GRF ein ungewollte Absinken oder Einknicken zu vermeiden.

Eine schlagartige Erhöhung des Widerstandes nach Erreichen bestimmter Schwellwerte wird häufig als nicht angenehm empfunden. Daher ist es vorgesehen, dass der Widerstand nach Erreichen eines Schwellwertes für die Bodenreaktionskraft GRF kontinuierlich erhöht wird. Typischerweise würde die Erhöhung des Widerstandes zwischen 20% und 30% des Körpergewichtes durchgeführt, die Reduktion zwischen 20% und 10% des Körpergewichtes. Liegt jedoch ein zu großer Kniewinkel $\alpha_K$ oder Inertialwinkel $\alpha_T$ vor oder wird das Gelenk 4 bewegt, kann davon ausgegangen werden, dass keine Stehsituation vorliegt, so dass eine Reduzierung des Widerstandes, ggf. bis auf einen Ausgangswiderstand vorgenommen werden kann. Befindet sich das Oberschenkelteil 1 in einer geneigten Stellung, also in einem im Wesentlichen waagerechten Zustand, so dass der Inertialwinkel $\alpha_T$ zwischen beispielsweise 70° und 110° befindlich ist, soll eine Erhöhung des Widerstandes ausgeschlossen werden. Gegebenenfalls kann nach Ablauf einer vorgegebenen Zeit sogar eine Reduzierung des Widerstandes der Widerstandeinrichtung unter den Standphasenwiderstand erfolgen, da dann angenommen werden kann, dass der Prothesennutzer sitzt.

Zur Bestimmung der Inertialwinkelgeschwindigkeit wird die Veränderung des Inertialwinkels $\alpha_1$ über die Zeit ermittelt, so dass sich eine Winkelgeschwindigkeit $\omega_1$ ergibt, die mit Betrag und Richtung ermittelt werden kann. Liegt nun ein bestimmter Inertialwinkel $\alpha_1$ und eine bestimmte Inertialwinkelgeschwindigkeit $\omega_1$ vor, kann davon ausgegangen werden, dass eine Bewegungssituation vorliegt, also keine Stehsituation, in der eine Verriegelung oder Blockade des Kniegelenkes auszuführen wäre.

Bei dem vorliegenden Verfahren ist es vorgesehen, dass sofort nach einer Belastung, die einen bestimmten Schwellwert überschreitet, ohne Zeitverzögerung eine Sperre eintritt, so dass kein gesondertes Aktivieren einer Widerstandserhöhung durch besondere Bewegungen, die nicht einem normalen Bewegungsablauf entsprechen, eingeleitet werden muss. Über die Bodenreaktionskraft GRF wird ein Belastungssignal bereitgestellt, das die Größe der Belastung repräsentiert, wobei die Bodenreaktionskraft GRF ausreicht, um die Stehfunktion zu aktivieren. Weitere Größen wie Momente oder Inertialwinkel können ergänzend, also zur Absicherung der Entscheidung, ob tatsächlich ein Stehzustand oder eine Standphase vorliegt, herangezogen werden. Mit dem beanspruchten Verfahren ist es möglich, das Knie oder ein anderes Gelenk in gebeugter Stellung oder unter beugenden Kräften zu sperren, wobei vorteilhafterweise die Sperrung nur bei einem leicht gebeugten Knie einsetzt, beispielsweise ab einer Kniebeugung von 4° als Kniewinkel.

## Patentansprüche

1. Verfahren zur Steuerung eines künstlichen orthetischen oder prothetischen Kniegelenkes (4) mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Kniegelenkes (4) einer Steuereinrichtung über Sensoren Zustandsinformationen bereitgestellt werden, wobei der jeweilige Widerstand auf der Grundlage empfangener Sensordaten und der Auswertung der Sensordaten verändert wird, indem der Aktuator entsprechend der Auswertung aktiviert wird, **dadurch gekennzeichnet, dass** der Widerstand in der Standphase oder während des Stehens von einem Ausgangswert in Abhängigkeit von der Bodenreaktionskraft (GRF) bis zu einer Sperre des Kniegelenkes (4) erhöht wird.

2. Verfahren nach Anspruch 1, **dadurch gekennzeichnet, dass** der Widerstand während der Standphase bei zunehmender Belastung kontinuierlich erhöht wird.

3. Verfahren nach Anspruch 1 oder 2, **dadurch gekennzeichnet, dass** der Widerstand bei Erreichen oder Überschreiten eines Schwellwertes erhöht wird.

4. Verfahren nach einem der voranstehenden Ansprüche, **dadurch gekennzeichnet, dass** der Widerstand in Abhän- gigkeit von einem Gelenkwinkel $\alpha_k$ und/oder Inertialwinkel $\alpha_t$ einer Gelenkkomponente erhöht wird.

5. Verfahren nach einem der voranstehenden Ansprüche, **dadurch gekennzeichnet, dass** der Widerstand in Abhängigkeit von dem Abstand der Bodenreaktionskraft (GRF) zu einem Bezugspunkt an einem Anschlussteil (1, 2) an dem Kniegelenk (4) oder in Abhängigkeit von einem Moment um den Bezugspunkt erhöht oder gesperrt wird.

6. Verfahren nach einem der voranstehenden Ansprüche, **dadurch gekennzeichnet, dass** der Widerstand nach dem Erhöhen in Abhängigkeit von einem Inertialwinkel $\alpha_t$, einer Änderung des Inertialwinkels $\alpha_t$ und/oder der Inertial- winkelgeschwindigkeit eines Anschlussteils (1, 2) des Kniegelenks (4) reduziert wird.

7. Verfahren nach Anspruch 4 oder 6, **dadurch gekennzeichnet, dass** der Inertialwinkel $\alpha_t$ des Anschlussteils (1, 2) unmittelbar oder aus dem Inertialwinkel $\alpha_t$ eines anderen Anschlussteils (1, 2) und einem Gelenkwinkel $\alpha_k$ ermittelt wird.

8. Verfahren nach einem der voranstehenden Ansprüche, **dadurch gekennzeichnet, dass** der Widerstand nach dem Erhöhen in Abhängigkeit von einem gemessenen Gelenkwinkel $\alpha_k$ und/oder einer gemessenen Gelenkwinkelgeschwindigkeit reduziert wird.

9. Verfahren nach einem der voranstehenden Ansprüche, **dadurch gekennzeichnet, dass** der Widerstand nach dem Erhöhen in Abhängigkeit von einem Abstand (A) und/oder der Änderung des Abstandes (A) der Bodenreaktionskraft (GRF) zu einem Bezugspunkt an einem Anschlussteil (1, 2) an dem Kniegelenk (4) wieder auf den Ausgangswert reduziert wird.

10. Verfahren nach einem der voranstehenden Ansprüche, **dadurch gekennzeichnet, dass** die Kriterien zur Erhöhung und/oder Verringerung des Widerstandes in einem Kennfeld direkt miteinander kombiniert werden.

11. Verfahren nach einem der voranstehenden Ansprüche, **dadurch gekennzeichnet, dass** mehrere Steueralgorithmen vorhanden sind, die auf der Grundlage von Messwerten unterschiedlicher Einrichtungen zur Erfassung von Winkeln und Kräften arbeiten, so dass bei Ausfall einer Einrichtung zur Erfassung von Winkeln und Kräften die übrigen Messwerte zur Steuerung der Veränderung des Streck- und/oder Beugewiderstandes verwendet werden.

12. Vorrichtung zur Durchführung des Verfahrens nach einem der voranstehenden Ansprüche, mit einer einstellbaren Widerstandseinrichtung, die zwischen zwei gelenkig aneinander gelagerten Komponenten eines künstlichen orthetischen oder prothetischen Kniegelenkes (4) angeordnet ist, mit einer Steuereinrichtung und Sensoren, die Zustandsinformationen der Vorrichtung erfassen, **dadurch gekennzeichnet, dass** eine Einstellvorrichtung vorgesehen ist und dass über die Einstellvorrichtung eine belastungsabhängige Widerstandsänderung aktivierbar und/oder deaktivierbar ist.

**Claims**

1. A method for controlling an orthotic or prosthetic knee joint (4) with a resistance device, which is assigned at least one actuator by way of which the bending and/or stretching resistance is changed in dependence on sensor data, information pertaining to the state being provided by way of sensors during the use of the joint, wherein, in the standing phase or when standing, the respective resistance is changed on the basis of received sensor data and the evaluation of the sensor data by activating the actuator according to the evaluation, **characterized in that** the resistance is increased from an initial value up until a locking of the knee joint (4) in dependence on the measured ground reaction force (GRF).

2. The method as claimed in claim 1, **characterized in that** the resistance is increased continuously during the standing phase when there is increasing loading.

3. The method as claimed in claim 1 or 2, **characterized in that** the resistance is increased when a threshold value is reached or exceeded.

4. The method as claimed in one of the preceding claims, **characterized in that** the resistance is increased in dependence on a joint angle $\alpha_k$ and/or inertial angle $\alpha_t$ of a joint component.

5. The method as claimed in one of the preceding claims, **characterized in that** the resistance is increased or locked in dependence on the distance of the ground reaction force (GRF) from a reference point on a connection part (1, 2) on the knee joint (4) or in dependence on a torque about the reference point.

6. The method as claimed in one of the preceding claims, **characterized in that**, after increasing, the resistance is reduced in dependence on an inertial angle $\alpha_t$, a changing of the inertial angle $\alpha_t$ and/or the inertial angle velocity of a connection part (1, 2) of the knee joint (4).

7. The method as claimed in claim 4 or 6, **characterized in that** the inertial angle $\alpha_t$ of the connection part (1, 2) is determined directly or from the inertial angle $\alpha_t$ of another connection part (1, 2) and a joint angle $\alpha_k$.

8. The method as claimed in one of the preceding claims, **characterized in that**, after the increasing, the resistance is reduced in dependence on a measured joint angle $\alpha_k$ and/or a measured joint angle velocity.

9. The method as claimed in one of the preceding claims, **characterized in that**, after the increasing, the resistance is reduced again to the initial value in dependence on a distance (A) and/or the changing of the distance (A) of the ground reaction force (GRF) from a reference point on a connection part (1, 2) on the knee joint (4).

**10.** The method as claimed in one of the preceding claims, **characterized in that** the criteria for increasing and/or reducing the resistance are combined with one another directly in a characteristic diagram.

**11.** The method as claimed in one of the preceding claims, **characterized in that** there are a number of control algorithms, which operate on the basis of measured values of different devices for detecting angles and forces, so that, in the case of failure of one device for detecting angles and forces, the other measured values are used for controlling the changing of the stretching and/or bending resistance.

**12.** A device for carrying out the method as claimed in one of the preceding claims, with a settable resistance device, which is arranged between two components of an artificial orthotic or prosthetic knee joint (4) that are arranged one against the other in a jointed manner, with a control device and sensors that detect information pertaining to the state of the device, **characterized in that** a setting device is provided and **in that** a loading-dependent change in resistance can be activated and/or can be deactivated by way of the setting device.

**Revendications**

**1.** Procédé pour la commande d'une articulation artificielle orthétique ou prothétique du genou (4) comprenant un système à résistance auquel est associé au moins un actionneur au moyen duquel la résistance à la flexion et/ou à l'extension est modifiée en fonction de données sensorielles, dans lequel pendant l'utilisation de l'articulation du genou (4) des informations d'état sont mises à disposition d'un système de commande via des capteurs, dans lequel la résistance respective est modifiée sur la base de données sensorielles reçues et de l'analyse des données sensorielles, en activant l'actionneur en correspondance de l'analyse, **caractérisé en ce que** la résistance est augmentée pendant la phase d'immobilisation ou pendant la station debout depuis une valeur de départ en fonction de la force de réaction au sol (GRF) jusqu'à un blocage de l'articulation du genou (4).

**2.** Procédé selon la revendication 1, **caractérisé en ce que** la résistance est augmentée en continu pendant la phase d'immobilisation lorsque la charge est croissante.

**3.** Procédé selon la revendication 1 ou 2, **caractérisé en ce que** la résistance est augmentée si l'on atteint ou si l'on dépasse une valeur seuil.

**4.** Procédé selon l'une des revendications précédentes, **caractérisé en ce que** la résistance est augmentée en fonction d'un angle $\alpha k$ de l'articulation et/ou d'un angle d'inertie $\alpha t$ d'une composante de l'articulation.

**5.** Procédé selon l'une des revendications précédentes, **caractérisé en ce que** la résistance est augmentée ou bloquée en fonction de la distance de la force de réaction au sol (GRF) jusqu'à un point de référence sur une pièce de raccordement (1, 2) sur l'articulation du genou (4) ou en fonction d'un couple autour du point de référence.

**6.** Procédé selon l'une des revendications précédentes, **caractérisé en ce qu'**après une augmentation en fonction d'un angle d'inertie $\alpha_t$, d'une modification de l'angle d'inertie $\alpha t$ et/ou de la vitesse de l'angle d'inertie d'une pièce de raccordement (1, 2) de l'articulation du genou (4), la résistance est réduite.

**7.** Procédé selon la revendication 4 ou 6, **caractérisé en ce que** l'angle d'inertie $\alpha t$ de la pièce de raccordement (1, 2) est déterminé directement ou à partir de l'angle d'inertie $\alpha t$ d'une autre pièce de raccordement (1, 2) et d'un angle d'articulation $\alpha k$.

**8.** Procédé selon l'une des revendications précédentes, **caractérisé en ce qu'**après une augmentation en fonction d'un angle d'articulation mesuré $\alpha k$ et/ou d'une vitesse angulaire d'articulation mesurée, la résistance est réduite.

**9.** Procédé selon l'une des revendications précédentes, **caractérisé en ce qu'**après augmentation en fonction d'une distance (A) et/ou de la modification de la distance (A) de la force de réaction au sol (GRF) jusqu'à un point de référence sur une pièce de raccordement (1, 2) sur l'articulation du genou (4), la résistance est à nouveau réduite à la valeur de départ.

**10.** Procédé selon l'une des revendications précédentes, **caractérisé en ce que** les critères pour augmenter et/ou pour réduire la résistance sont combinés directement les uns avec les autres dans un champ de valeurs caractéristiques.

**11.** Procédé selon l'une des revendications précédentes, **caractérisé en ce qu'**il est prévu plusieurs algorithmes de commande, qui fonctionnent sur la base de valeurs de mesures provenant de systèmes différents pour la détection d'angles et de forces, de sorte qu'en cas de défaillance d'un système pour la détection d'angles et de forces on utilise les autres valeurs de mesures pour la commande de la modification de la résistance à l'extension et/ou à la flexion.

**12.** Appareil pour la mise en oeuvre du procédé selon l'une des revendications précédentes, comprenant un système de résistance réglable, qui est agencé entre deux composants, montés de façon articulée l'un par rapport à l'autre, d'une articulation artificielle orthétique ou prothétique du genou (4), comprenant un système de commande et des capteurs qui déterminent des informations d'état de l'appareil, **caractérisé en ce qu'**il est prévu un dispositif de réglage, et **en ce qu'**il est possible d'activer et/ou de désactiver une modification de la résistance en fonction de la charge au moyen du dispositif de réglage.

Fig. 1

Fig. 2

EP 2 498 724 B1

Fig. 3

$$a = \frac{M}{F_{AX}}$$

Fig. 4

$$b = \frac{M_1 + \dfrac{M_2 - M_1}{l_2 - l_1} \cdot (x - l_1)}{F_{AX}}$$

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008008284 A1
- DE 102006021802 A1
- DE 102007053389 A1
- DE 60309685 T2
- US 20080228287 A1